(19) Europäisches Patentamt

European Patent Office

Office européen des brevets

(11) **EP 0 911 043 B1**

(12) **EUROPÄISCHE PATENTSCHRIFT**

(45) Veröffentlichungstag und Bekanntmachung des Hinweises auf die Patenterteilung:
**07.07.2004   Patentblatt 2004/28**

(51) Int Cl.⁷: **A61M 1/16**

(21) Anmeldenummer: **98120055.3**

(22) Anmeldetag: **23.10.1998**

(54) **Vorrichtung zur Messung von Leistungsparametern von Stoff- und Energieaustauschmodulen**

Device for measuring performance parameters of substance and energy exchange modules

Dispositif pour déterminer des paramètres de performance de modules d'échangeurs de substances et d'énergie

(84) Benannte Vertragsstaaten:
**AT BE CH DE DK ES FI FR GB GR IE IT LI NL PT SE**

(30) Priorität: **27.10.1997   DE 19747360**

(43) Veröffentlichungstag der Anmeldung:
**28.04.1999   Patentblatt 1999/17**

(60) Teilanmeldung:
**04011484.5**

(73) Patentinhaber: **Fresenius Medical Care Deutschland GmbH**
**61352 Bad Homburg v.d.H. (DE)**

(72) Erfinder:
- **Polaschegg, Hans-Dietrich**
  **9231 Köstenberg (AT)**
- **Dr Helmut Steil**
  **61231 Bad Nauheim (DE)**

(74) Vertreter: **Oppermann, Frank, Dipl.-Ing. et al**
**Luderschmidt, Schüler & Partner GbR,**
**Postfach 39 29**
**65029 Wiesbaden (DE)**

(56) Entgegenhaltungen:
**EP-A- 0 547 025          EP-A- 0 658 352**
**WO-A-98/32476**

Anmerkung: Innerhalb von neun Monaten nach der Bekanntmachung des Hinweises auf die Erteilung des europäischen Patents  kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Hämodialyse hat sich in den letzten 30 Jahren zu einer lebensrettenden Einrichtung für mehrere hunderttausend Patienten weltweit entwickelt.

Da es sich dabei um eine chronische Behandlung handelt, sind die Kosten für die Volkswirtschaft in diesem Zeitraum gewaltig angestiegen.

Um die Behandlung für die weiter wachsende Zahl von Patienten in den Industrieländern zu sichern und in den Schwellenländern überhaupt erst zu ermöglichen, gilt es. die Qualität des Verfahrens zu optimieren und andererseits die Kosten zu senken. Die Optimierung des Verfahrens ist dabei gleichzeitig ein wesentlicher Kostenfaktor, da ein gut behandelter Patient weniger morbide ist und daher weniger Pflege braucht.

Im Rahmen der NCDS National Cooperative Dialysis Study) wurde in den USA die Morbidität eines großen Patientenkollektivs in Abhängigkeit von der "Dialysedosis" untersucht. Gotch und Sargent ( Kidney International 28, 526-534,1985) fanden für die ermittelten Resultate eine einfache Erklärung: Die Morbidität fällt von einem hohen Wert auf einen niedrigen konstanten Wert ab wenn der Wert für K.t/V von 0,8 auf >= 1 ansteigt. K ist dabei die effektive Clearance für Harnstoff, t die Behandlungszeit und V das Gesamtkörperwasser.

Die Hypothese, daß Morbidität und Mortalität mit der durch den Kt/V- Parameter für Harnstoff beschriebenen Parameter zusammenhängt hat sich zwar für den einheitlichen Behandlungsmarkt der USA bewährt, nicht jedoch die Interpretation der NCDS Daten durch Sargent und Gotch. Neuere Daten weisen darauf hin, daß die Mortalität bis zu einem Kt/V von 1.5 weiter abnimmt. Darüber hinaus liegen gesicherte Daten mangels ausreichender Patientenzahlen nicht vor (Dazu siehe: Parker Thomas F. Short-Time Dialysis Should Be Used Only With Great Caution. Seminars in Dialysis 1993;6:164-167, Hakim RM, Breyer J, Ismail N, Schulman G. Effects of dose of dialysis on morbidity and mortality. Am J Kidney Dis 1994;23:661-9, Parker TF, Husni L, Huang W, Lew N, Lowrie EG. Survival of hemodialysis patients in the united states is improved with a greater quantity of dialysis. Am J Kidney Dis 1994;23:670-80 und andere).

Diese Erkenntnisse haben dazu geführt, daß in den USA Richtlinien erstellt wurden (DOQI guidelines) die ein Mindestdosis von Kt/V=1.2 bzw. 1.4 für Diabetiker fordert. Diese Richtlinien werden nun von der Aufsichtbehörde als relevant anerkannt und es muß durch geeignete Verfahren die Einhaltung dieser Mindestanforderungen nachgewiesen werden.

Eine Möglichkeit dazu besteht in der Ermittlung der effektiven Clearance, der Behandlungszeit und des Gesamtkörperwassers. Die Behandlungszeit ist trivial ermittelbar, das Gesamtkörperwasser kann mit bekannten Methoden, z.B. der Bioimpedanztechnik oder mit Hilfe des Harnstoffmodells ermittelt werden. Die effektive Clearance ist mit konventionellen Methoden schwierig zu ermitteln, jedoch wurde vom Erfinder in der DE3938662 ein Verfahren zur invitro Bestimmung der effektiven Elektrolytdialysance, die im Rahmen der Meßgenauigkeit gleich der effektiven Clearance für Harnstoff ist, angegeben. In-vitro und in-vivo Experimente haben inzwischen gezeigt, daß dieses Verfahren in der Praxis durchführbar ist. Es wird auf Tagungen, wie z.B. dem EDTA Kongreß in Genf 1997 und dem ASN Kongreß 1997 in San Antonio seitens der Industrie dafür geworben.

Die oben gemachte Annahme, daß Elektrolytdialysance annähernd gleich der Harnstoff Clearance ist, trifft allerdings nur zu, wenn es sich beim Elektrolyt um eine Gemisch handelt, das üblicherweise als "Säurekonzentrat" bezeichnet wird und im wesentlichen aus Chloriden besteht. Wird, z.B. die Konzentration des gesamten Dialysats oder nur der Bicarbonatkomponente verändert, so ist die Übereinstimmung geringer.

Bei dem in der DE3938662 beschriebenen Verfahren wird der Elektrolyttransfer bei zwei (oder mehr) Eingangselektrolytkonzentrationen gemessen und daraus die Dialysance berechnet. Eingangs- und Ausgangskonzentration müssen dabei über einen Zeitraum von etwa 1- 5 Minuten konstant gehalten werden. Schwankungen führen auf Grund der Zeitkonstante der Messung zu einem Fehler. Die Zeitkonstante entsteht durch das Füllvolumen des Dialysators und führt dazu, daß nach Anlegen einer Sprungfunktion am Eingang, die Ausgangskonzentration sich nur verzögert und langsam einstellt. Eine solche Kurve ist in der Arbeit des Erfinders: Polaschegg HD, Levin NW. Hemodialysis Machines and Monitors.. Jacobs C, Kjellstrand CM, Koch KM, Winchester JF, editors. Replacement of renal function by dialysis. 4th ed.. Kluwer academic publishers, 1996:333-79. Das hier zitierte Buch enthält auch alle weitem, für das Verständnis dieser Ausführungen notwendigen Informationen zum Stand der Technik. Die Veränderung der Elektrolytkonzentration erfolgt in der Regel automatisch durch Änderung des Mischverhältnisses zwischen Konzentrat und Wasser im Dialysegerät. Da nun die Einstellung einer neuen Dialysierflüssigkeitskonzentration ebenfalls einer Zeitkonstante unterworfen ist, dauert die gesamte Messung mehrere Minuten und ist mit einem beträchtlichen Elektrolyttransfer verbunden, der anschließend durch eine gesteuerte Zufuhr oder Abfuhr kompensiert werden kann. Das Verfahren ist damit in der Praxis nur für neu konstruierte Geräte geeignet, eine Nachrüstung für bestehende Geräte ist aufwendig und wird auch nicht angeboten. Wegen der relativ langen Meßzeit, kann mit diesem Verfahren nur die effektive Clearance, nicht aber die Dialysator Clearance gemessen werden. Die beiden Werte unterscheiden sich durch den Einfluß der Rezirkulation im Blutzugang bzw. im Kreislaufsystem, ein für sich interessanter Parameter. Schließlich ist das Verfahren, sofern die Veränderung der Dialysierflüssigkeitskonzentration durch das Mischsystem des Dialysegerätes erfolgt, nur für Einzelplatzdialysegeräte, nicht aber für zentralversorgte Dialysegeräte geeignet.

Somit ergeben sich für das vom Erfinder beschriebene und bereits in Anwendung befindliche Verfahren folgende Einschränkungen bzw. Nachteile:

Die Messung dauert relativ lange und ist mit einem nicht zu vernachlässigendem Elektrolyttransfer verbunden. Das Verfahren ist nicht für die Messung von Stoffen, die nicht im Dialysat enthalten sind, geeignet (z.B. Kreatinin, Phosphate). Das Verfahren kann nicht zwischen Dialysator Clearance und effektiver Clearance unterscheiden.

Die vorliegende Erfindung hat die Aufgabe, diese Nachteile zu verringern und darüber hinaus eine Vorrichtung anzugeben, die leicht und ohne Eingriff in die elektronische Steuerung an bestehende Geräte angepaßt werden kann und die darüber hinaus für andere Substanzen gleichermaßen geeignet ist. Bei der erfindungsgemäßen Vorrichtung wird eine vorbestimmte Substanzmenge dem Dialysatkreislauf stromauf des Dialysators zugefügt und die den Dialysator stromab im Dialysatkreislauf verlassende Menge bestimmt und daraus die Dialysance bzw. Clearance für die zugeführte Substanz berechnet.

Die Zufuhr der Substanz kann auf unterschiedliche Weise erfolgen, auch kann statt einer Zufuhr eine Verdünnung erfolgen. Es kann stromauf des Dialysators ein flüssiges Konzentrat einer einzigen Substanz (z.B. NaCl oder Kreatinin) zugespritzt werden. Alternativ kann auch einfach Wasser zugespritzt werden. Das Zuspritzen kann manuell mit einer Spritze oder halbautomatisch mit einer federbelasteten Spritze oder automatisch mit Hilfe einer Pumpe erfolgen. Auch kann die in der Regel im Dialysegerät eingebaute Konzentratförderpumpe kurzzeitig mit einer höheren Rate betrieben werden um einen Elektrolytbolus zu erzeugen. Alternativ zur Zugabe einer vorbekannten Stoffmenge kann man auch eine nicht vorbekannte Stoffmenge zugeben, diese aber durch einen Sensor, der stromauf des Dialysators angebracht ist bestimmen. Man kann ferner eine nicht genau bekannte, aber wiederholbar zuspritzbare Menge durch eine erste Boluszuspritzung kalibrieren um dann mit einer zweiten Zuspritzung die eigentliche Messung vorzunehmen. Dazu wird die Kalibrierzuspritzung entweder stromab des Dialysators aber stromauf des Sensors vorgenommen oder aber der Dialysatkreislauf wird in den Bypass geschaltet und die Kalibrations-Zuspritzung erfolgt an der gleichen Stelle stromauf des Dialysators wie die Zuspritzung für die eigentliche Messung.

Anstelle der Zuspritzung einer flüssigen Substanz kann eine Zugabe einer Stoffmenge auch durch die kurzzeitige Durchleitung der Dialysierflüssigkeit durch eine Pulverkartusche oder einen Pulverbeutel erfolgen.

Die Erfindung wird nun an Hand einer mathematischen Ableitung sowie an Hand von Abbildungen zu verschiedenen Ausführungsformen näher erläutert.

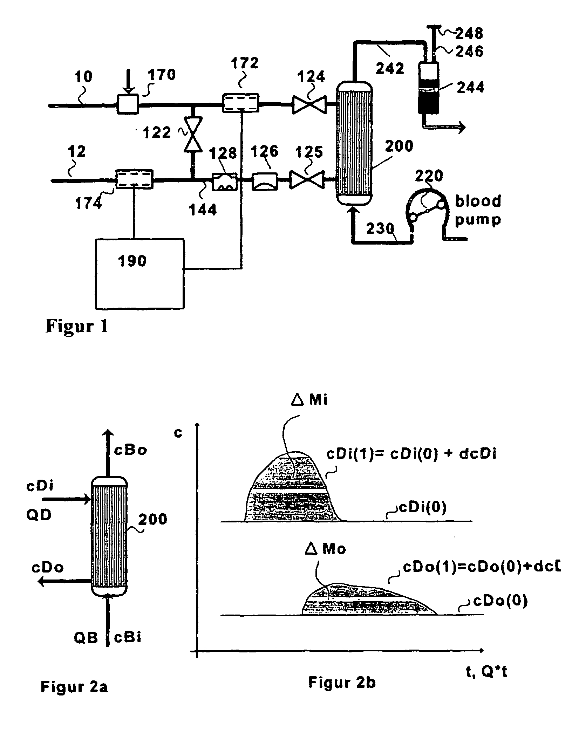

Es zeigt **Figur 1** einen Ausschnitt aus dem Flüssigkeitskreislauf eines herkömmlichen Dialysegerätes. 10 ist die Dialysatzuleitung von einer nicht näher dargestellten Dialysatquelle. Dabei kann es sich um das Mischsystem eines Einzelplatzdialysegerätes, aber auch um eine zentrale Dialysatversorgung handeln. In diese Leitung ist eine Zuspritzstelle oder Zufuhranschluß 170 eingefügt, über die die Stoffmenge zur Clearancemessung zugeführt werden kann. In der Leitung folgt ein erster Sensor zur Clearancemessung 172, der optional ist sowie ein erstes Dialysatorventil 124. 200 ist der Dialysator, der durch eine semipermeable Membran in ein Blut und Dialysatteil getrennt wird (nicht näher dargestellt). Verbrauchte Dialysierflüssigkeit verläßt den Dialysator durch die Leitung 144. Sie strömt zunächst durch das zweite Dialysatorventil 125, einen Drucksensor 126 und einen Blutleckdetektor 128 und gelangt schließlich zum zweiten Sensor zur Clearancemessung 174 und weiter durch die Leitung 12 zum nicht näher dargestellten Abfluß. Von der Leitung 10 zweigt eine Bypassleitung mit Bypassventil 122 zur Leitung 144 ab. Die Zuspritzstelle 170 ist vorteilhafterweise, nicht aber zwingend stromauf des Bypassventils angebracht, der zweite Sensor zur Clearancemessung 174 ist vorteilhafterweise stromab vom Bypassventil in der Abflußleitung angebracht. Der Sensor 174 und der optionale Sensor 172 ist jeweils mit einer Auswerteeinheit 190 verbunden.

**Figur 2a** zeigt den Dialysator 200 mit Blut sowie Dialysat Zu- und Ableitungen und die in der mathematischen Ableitung verwendeten Bezeichnungen. Es ist QB der Blutfluß und QD der Dialysatfluß. cBi ist die Blutkonzentration am Eingang, cBo die Blutkonzentration am Ausgang der Blutseite des Dialysators. Auf der anderen Seite der Membran fließt das Dialysat, in der Regel im Gegenstrom. Dabei ist cDi die Konzentration am Dialysateingang und cDo die Konzentration am Ausgang.

Zur folgenden Ableitung soll bezüglich des Standes der Technik ausdrücklich auf die schon erwähnte Patentschrift DE 3938662 sowie die Publikation des Erfinders:" Polaschegg HD. Automatic, noninvasive intradialytic clearance measurement. Int J Artif Organs 1993;16:185-191" hingewiesen werden.

Die Dialysance D kann aus dem Dialysatfluß QD, den Dialysatkonzentrationen Cdi und Cdo sowie der Blutkonzentration cBi wie folgt errechnet werden:

$$D = QD * \frac{CDi - CDo}{CBi - CDi} \qquad (1)$$

Die Konzentrationen vor der Stoffzugabe werden mit dem Index 0 bezeichnet und die Konzentration während des Stoffbolus mit dem Index 1. Für die folgende, vereinfachte Ableitung wird ferner angenommen, daß der Dialysatfluß und die Dialysance(und damit auch der Blutfluß) während der Messung konstant bleibt und keine Ultrafiltration stattfindet. Die erfindungsgemäße Methode ist jedoch auch bei gleichzeitiger Ultrafiltration anwendbar.

Somit gilt:

$$D^0=D^1=D \ und \ QD^0=QD^1=QD \tag{2}$$

Es wird ferner eine weitere Annahme gemacht, die für die erfindungsgemäße Messmethode zutrifft, nicht jedoch für die in der DE3938662 beschriebene. Es wird angenommen, daß während der Messung sich die Bluteingangskonzentration nicht ändert.

$$cBi^0=cBi^1 \tag{3}$$

1, mit 2 und 3 läßt sich nun für beide Indizes wie folgt umwandeln:

$$D^*CBi-D^*cDi^0-QD^*(cDi^0-cDo^0)$$

$$D^*CBi-D^*cDi^1=QD^*(cDi^1-cDo^1) \tag{4}$$

Die untere Formel aus 4 wird nun verwendet um D*CBi in der oberen Formel zu ersetzen. Damit wird die unbekannte Größe cBi eliminiert. Es ergibt sich:

$$D^*(cDi^1-cDi^0)=QD^*(cDi^1-cDi^0)-QD^*(cDo^1-cDo^0) \tag{5}$$

Und schließlich ergibt sich:

$$D=\frac{QD^*(cDi^1-cDi^0)-QD^*(cDo^1-cDo^0)}{cDi^1-cDi^0} \tag{6}$$

Beide Seiten werden nun durch QD dividiert:

$$\frac{D}{QD}=\frac{QD^*(cDi^1-cDi^0)-QD^*(cDo^1-cDo^0)}{QD^*(cDi^1-cDi^0)} \tag{7}$$

Unter der Annahme, daß sich die Konzentration im zufließenden Dialysat nicht ändert, kann man $cDi^1$ als Überlagerung einer konstanten Konzentration $cDi^0$ und einer Boluskonzentration dcDi ansehen, wie dies in **Figur 2b** graphisch dargestellt ist.

$$cDi^1=cDi^0+dcDi \ , \ cDo^1=cdo^0+dcDo \tag{8}$$

Mit 7 und 8 erhält man weiter:

$$\frac{D}{QD}=\frac{QD^*(dcDi)-QD^*(dcDo)}{QD^*(dcDi)} \tag{9}$$

Nun ergibt die Integration über dcDi bzw. dcDo über die Zeit multipliziert mit QD gerade die Stoffmenge die stromauf zugeführt wurde bzw. die stromab den Dialysator wieder verläßt:

$$\Delta M1=QD^*\int dcDi^*dt \ , \ \Delta M2=QD^*\int dcDo^*dt \tag{10}$$

Das Integrationsintervall muß dabei so lange gewählt werden, bis der Bolus am Ausgang bis auf einen vernachlässigbaren Anteil abgeklungen ist.

4

Mit 9 und 10 Ergibt sich somit:

$$D=QD*\frac{\Delta Mi-\Delta Mo}{\Delta Mi} \tag{11}$$

Da die Stoffmenge $\Delta Mi$ vorgebbar ist, muß lediglich die Konzentration am Ausgang als Funktion der Zeit gemessen und darüber, nach Abziehen des Untergrundes das Integral gebildet werden. In der Praxis wird dies so verwirklicht, daß entweder kontinuierlich ein laufender Mittelwert der Ausgangskonzentration errechnet wird oder aber unmittelbar vor der Messung. Zu Beginn der Stoffzugabe wird entweder automatisch oder durch eine nicht näher dargestellte Eingabevorrichtung das Integrationsprogramm in der Auswerteeinheit 190 gestartet. Die Auswerteeinheit erhält aus der Steuereinheit des Dialysegerätes ferner ein dem Dialysatfluß proportionales Signal und über eine weitere Eingabeeinheit Informationen über die Stoffmenge.

Die Meßkonstanten zur Umsetzung des Sensorsignals in ein Konzentrationssignal sind entweder fest in der Auswerteeinheit 190 abgelegt oder können gleichermaßen durch eine Eingabeeinheit eingegeben werden. Solche Eingabeeinheiten sind Stand der Technik und brauchen daher nicht näher beschrieben werden. Digitale und analoge Eingabe, sowie Eingabe über Computer ist gleichermaßen möglich.

Die Steuereinheit errechnet nun aus den eingegeben Konstanten und der gemessenen Stoffmenge, die den Dialysator verläßt, entsprechend Gleichung 11 die Dialysance D und gibt diese auf einem Anzeigegerät wieder oder sendet die Information an einen externen Computer oder speichert diese.

Es wird darauf hingewiesen, daß die Formeln von Stoffmengen bzw. Konzentrationen ausgehen. In der Regel wird eine Stoffkonzentration indirekt, z.B. über die Leitfähigkeit, gemessen. Ist die Abhängigkeit zwischen Stoffkonzentration oder Stoffmenge und dem gemessenen physikalischen Parameter im relevanten Bereich nicht linear, so muß dies berücksichtigt werden. Dies ist z.B. der Fall, wenn die Leitfähigkeit zur Messung verwendet wird und dabei ein weiter Leitfähigkeitsbereich benutzt wird. Die entsprechenden Transferfunktionen können vorteilhafterweise direkt in der Auswerteeinheit 190 abgelegt werden. Alternativ können auch die Signale direkt an einen externen Computer weitergeleitet werden. Die Korrektur erfolgt dann durch ein geeignetes Programm.

Der optionale Sensor stromauf des Dialysators kann zur Bestimmung der Eingangsmenge herangezogen werden, wenn diese andernfalls nicht einfach vorgebbar ist, weil z.B. die Menge nicht hinreichend genau gesteuert werden kann.

**Zuspritzen von Wasser:** In einer besonderen Ausführungsform der Erfindung wird einfach Wasser zugespritzt. Damit läßt sich dann nicht mehr die Dialysance eines beliebigen Stoffes sondern nur mehr die Dialysance eines oder mehrerer Stoffe die im frischen Dialysat enthalten sind bestimmen. Als Eingangsstoffmenge $\Delta Mi$ wird dann das Volumen der zugegeben Wassermenge gewählt. Die Ausgangsstoffmenge $\Delta Mo$ wird aus dem Integral des nun relativ zur Basiskonzentration negativen Ausgangsbolus errechnet. Beim Zuspritzen von Wasser muß die Menge so bemessen werden, daß die Osmolarität der Dialysierflüssigkeit die Hämolysegrenze nicht unterschreitet.

Man kann über eine geeignete semipermeable Membran, z.B. eine Umkehrosmosemembran auch Wasser kurzzeitig entziehen. Dieser Fall kann dann wie die Zugabe eines Konzentrats behandelt werden.

**Störeinflüsse:** Durch die Injektion einer Stoffmenge wird der Dialysierflüssigkeitsfluß kurzzeitig erhöht. Vorteilhafterweise ist die Zugabestelle so weit vom Dialysator entfernt, daß der Stoffbolus den Dialysator erst erreicht, wenn der Fluß sich normalisiert hat. Bei sogenannten volumetrisch bilanzierenden Dialysesystemen kann die zugespritzte Menge nicht einfach eine gleich große Dialysierflüssigkeitsmenge in den Abfluß verdrängen, da es sich um ein geschlossenes System handelt. Diese Menge wird hingegen in den Patienten rückfiltriert bzw, bei andauernder Ultrafiltration wird diese für die Dauer des Bolus verringert. Dies kann eine geringfügige Störung der Ausgangsgrößen und somit eine Verletzung der Annahmen aus 6 und 6 bewirken. Dieser Einfluß, der abgeschätzt oder durch Experiment bestimmt werden kann, kann der Auswerteeinheit als Korrekturgröße eingegeben werden. Alternativ kann simultan zur Zuspritzung der Stoffmenge eine gleich große Flüssigkeitsmenge stromauf oder stromab des Dialysator abgezogen werden. Dies kann manuell oder automatisch mit einer Spritze oder Pumpe oder, gleichfalls automatisch, durch kurzzeitige Erhöhung der Ultrafiltrationsrate erfolgen. Alternativ kann eine Ausdehnungskammer- oder Beutel in den Dialysierflüssigkeitskreislauf integriert werden, um Druckstöße durch die Zugabe zu vermeiden.

Wird die Dialysance von Elektrolytlösungen bestimmt und nimmt man an, daß die Konzentration im Maximum um nicht mehr als 10% angehoben werden soll so ergibt sich eine momentane Flußerhöhung von 3 bis 5% bei Verwendung von Konzentraten mit 3 bis 5 molarer Konzentration. Diese Flußerhöhung ist in der Regel vernachlässigbar.

**Einfluß der Rezirkulation:** Die Ableitung der Formeln geht von einer konstanten blutseitigen Eingangskonzentration aus. Dementsprechend wird die Dialysatorclearance bestimmt. Die per Bolus zugegebene Stoffmenge wird zum Teil ins Blut transferiert und erhöht dort die ausgangsseitige Konzentration. Dieser blutseitige Bolus gelangt zum Blutzugang. Bei Vorliegen einer Rezirkulation in diesem Bereich, gelangt eine Teil dieses Bolus wieder auf die Bluteingangsseite des Dialysators. Ist zu diesem Zeitpunkt der Meßvorgang noch nicht abgeschlossen, so beeinflußt dies das Meßergebnis in der Weise, daß der Dialysatausgangsbolus vergrößert wird. Die nach 6 berechnete Clearance ist dann gegenüber der Dialysatorclearance erniedrigt. Man nennt diesen Wert die effektive Clearance. Der oben erwähnte Blutkonzentrationsbolus durchläuft den Blutkreislauf des Menschen, wobei ein Teil ebenfalls rezirkuliert wird und nach typisch 1 bis 2 Minuten wieder zum Blutzugang gelangt. Diese Rezirkulation wird Cardiopulmonäre Rezirkulation genannt. Durch entsprechend lange Integration kann auch dieser Einfluß mit erfaßt werden.

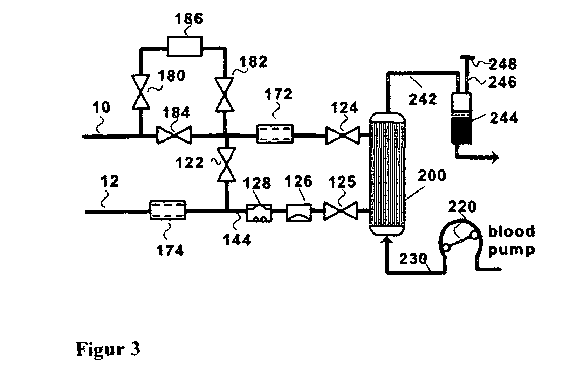

Durch geeignete Auslegung der Volumina im extrakorporalen System und dem Dialysierflüssigkeitskreislauf und mit Hilfe kurzer Zuspritzboli kann somit zumindest annähernd der Einfluß der Rezirkulation nicht nur integral sondern getrennt erfaßt werden. **Weitere Ausführungsformen:** Eine weitere bevorzugte Ausführungsform ist in Figur 3 dargestellt. 180, 182 und 184 sind Ventile und 186 ist ein Behälter, der entweder ein flüssiges oder ein pulverförmiges, granuliertes oder festes(gepreßtes) Konzentrat enthalten kann. Zur Erzeugung eines Stoffbolus wird der Dialysatfluß kurzzeitig durch den Behälter 186 geleitet. Dazu wird das Ventil 184 geschlossen und die Ventile 180 und 182 geöffnet. Bei diesem Vorgang wird der Dialysatfluß nicht verändert, es kommt somit zu keiner Störung wie weiter oben beschrieben. Das Ventil 180 kann als passives, federdruckbelastetes Ventil ausgelegt werden, daß bei einem bestimmten Überdruck, z.B. 0.2 bar öffnet. Das Ventil 182 kann als passives Rückschlagventil ausgeführt sein. Wird das Ventil 184 geschlossen, so steigt der Druck davor an und das Ventil 180 öffnet. Bei Öffnen des Ventils 184 schließt das Ventil 180 wieder.

Der Vorratsbehälter 186 kann ein flüssiges Konzentrat enthalten, daß durch das Schließen des Ventils 184 ausgeschwemmt wird. Man erhält dann einen äußert kurz andauernden Bolus. Da die Menge über das Volumen einfach bestimmbar ist, ist für dieses Verfahren ein Sensor stromauf des Dialysators (172) nicht erforderlich. Wird der Vorratsbehälter 186 mit einem festen Konzentrat gefüllt, daß sich in der Dialysierflüssigkeit nicht vollständig auflöst, so wird in der Regel die gelöste Menge nicht vorher bestimmbar sein. Dies kann aber durch den Sensor 172 bestimmt werden. Der Vorteil einer solchen Ausführungsform liegt darin, daß der Meßvorgang ohne Nachfüllung des Vorratsbehälters mehrmals wiederholt werden kann. Freilich kann der Vorratsbehälter auch mit einem Pulver gefüllt werden, daß beim Durchleiten des Dialysats vollständig gelöst wird. Diese Ausführungsform ist analog zur Füllung mit flüssigem Konzentrat.

**Sensoren**: Als Sensoren kommen alle Sensoren in Frage, die direkt oder indirekt die Konzentration eines Stoffes messen und eine ausreichende Zeitauflösung besitzen. Für Elektrolyte ist dies vorteilhafterweise ein Leitfähigkeitssensor. Wird stromauf ein reiner Stoff, z.B. NaCl oder NaHCO3, zugegeben, so kann man damit die Dialysance für diesen Stoff bestimmen, obwohl der Leitfähigkeitssensor nicht stoffspezifisch ist. Man kann damit z.B. die Dialysance für Natriumbicarbonat bestimmen, die wegen der Größe des Moleküls geringer ist als die des Harnstoffs, ein Umstand der gewöhnlich vernachlässigt wird. Werden mehrere spezifische Sensoren stromab eingesetzt, so kann durch Zugabe eines Stoffgemisches stromauf, gleichzeitig die Dialysance für mehrere Stoffe bestimmt werden. Zur Messung von Elektrolyten kommen hier ionensensitive Elektroden in Frage.

Zur Bestimmung der Dialysance von nicht leitfähigen Stoffen können optische Sensoren herangezogen werden. Z.B. Sensoren zur Lichtdrehung zur Bestimmung der Glucosekonzentration. Die Dialysance von Kreatinin und Harnstoff sowie verschiedener Aminosäuren kann durch optische Extinktion im ultravioletten Bereich gemessen werden.

In der Regel ist der Dialysierflüssigkeitsfluß bei Dialysegeräten genau bekannt. Wird die erfindungsgemäße Vorrichtung in einem Zusatzgerät eingesetzt so kann vorteilhafterweise ein Flußsensor vorgesehen werden, dessen Signal in das Auswertegerät eingespeist wird. In einer weiteren Ausführungsform kann auch noch das Signal der Ultrafiltrationspumpe eingespeist werden und die Dialysance entsprechend bekannter Näherungsformeln auf den Einfluß der Ultrafiltration korrigiert werden.

**Kalibrierung**: Zur Kalibrierung des Sensors, oder falls die zuzugebende Stoffmenge nicht genau bekannt ist, kann eine initiale Boluszugabe unmittelbar vor dem Sensor erfolgen. Dies entweder manuell oder automatisch durch Öffnen des Bypassventile 122 bei gleichzeitigem Schließen der Dialysatorventile 124 und 125. Bei automatischer Steuerung durch das Dialysegerät wird zunächst der Dialysierflüssigkeitskreislauf in den Bypass geschaltet und danach ein erster Bolus erzeugt. Dann wird der Bypass wieder geschlossen, ein stabiler Zustand abgewartet und ein weiterer Bolus erzeugt. Bei diesem Vorgang ist lediglich eine gute Reproduzierbarkeit, nicht jedoch eine präzise Vorgabe einer Stoffmenge oder ein Sensor stromauf des Dialysator erforderlich.

**Weitere Anwendungen:** Die erfindungsgemäße Vorrichtung könnte in-vivo im Prinzip auch durch Injektion auf der Blutseite durchgeführt werden, jedoch ist dies wegen des Risikos der bakteriellen Kontamination durch die Injektion unvorteilhaft. Der Nachweis auf der Dialysatseite kann dann wie beschrieben erfolgen. Der Nachweis auf der Blutseite ist in der Regel schwieriger, da in der Regel aus Kostengründen nicht-invasive Sensoren eingesetzt werden müssen und optische Verfahren durch den roten Blutfarbstoff, sowie die Plasmaproteine gestört werden.

Die Vorrichtung kann vorteilhafterweise in- vitro, z.B. zur Qualitätssicherung eingesetzt werden. Dabei kann Wasser auf beiden Seiten der Membran strömen und der Bolus auf der Dialysierflüssigkeitsseite, alternativ aber auch auf der Blutseite zugegeben werden, da bei diesen Messungen bakterielle Kontamination unbedeutend ist. Auch der Nachweis ist auf jeder der beiden Seiten möglich. Um Information über den Meßfehler zu erhalten kann man Sensoren stromab beider Kreise anordnen. Die zugeführte Menge muß dann gleich der Summe der an den beiden Ausgängen gemessenen sein.

Da ein einziger Meßvorgang nur wenige Minuten dauert, kann durch manuelles oder automatisches Zuspritzen einzelner Substanzen sehr rasch eine Meßreihe erstellt werden, z.B. die aller leitfähigen Substanzen, incl. Natriumphosphat. An Stelle von Wasser kann auch eine gepufferte Lösung verwendet werden, falls die Dialysance eines schwachen Elektrolyten gemessen werden soll. Über pH Sensoren kann die Dialysance von Säuren gemessen werden.

**Wärmetauscher:** Wärmetauscher werden durch die gleichen Formeln beschrieben, wie Dialysatoren. In der Tat sind die Gleichungen für den Dialysator analog zu den älteren Formeln für Wärmetauscher hergeleitet worden. Durch Zuspritzen von heißem oder kaltem Wasser auf einer Seite und Messen der Temperatur auf der anderen Seite kann somit der Wärmetransferkoeffizient analog zur Bestimmung der Dialysance bestimmt werden.

**Ausführung der Zuspritzstelle:** Die Einrichtung zur Zuführung der Stoffmenge stromauf des Dialysator kann als Zuspritzstelle mit Septum oder als Ventil ausgeführt sein. Geeignet sind auch Probennahmeventile, die entweder manuell geöffnet werden oder automatisch bei Einführen eines Luer connectors öffnen.

**Zugeführte Stoffmenge:** Das Volumen der Lösung mit der der Stoff zugeführt wird, soll möglichst klein gehalten werden um Störungen durch Veränderung des Dialysierflüssigkeitsflusses gering zu halten. Dementsprechend soll die Konzentration möglichst hoch sein. Die absolute Stoffmenge richtet sich nach der Auflösung des Sensors und darf jedenfalls physiologische Grenzen nicht überschreiten. Auch darf die resultierende Spitzenkonzentration physiologische Grenzen nicht über- oder unterschreiten. Für Elektrolyte ergibt sich folgende Abschätzung: Die normale Elektrolytkonzentration im Dialysat liegt bei 150 mmol/l, Konzentrate sind bis zu 5 molar herstellbar. Für einen Bolus von typisch 10% über der normalen Konzentration und einer Dauer von 1 Minute ist eine Stoffmenge von 7.5 mmol bei einem Dialysatfluß von 500 ml/min erforderlich. Dies entspricht einem Volumen von 7.5 mmol/ 5000 mmol/l = 1.5 ml. Wird Wasser eingespritzt so muß zur Absenkung der Konzentration um 10% über 1 Minute etwa 50 ml eingespritzt werden. Der Einspritzvorgang selbst kann schneller erfolgen. Auf Grund der ungleichmäßigen Strömung im Dialysator verteilt sich der Bolus und es wird somit die Spitzenkonzentration reduziert.

| Liste der Bezeichnungen | |
| --- | --- |
| Referenznummer | Bezeichnung |
| 10 | Dialysatzuleitung von einer nicht näher dargestellten Dialysierflüssigkeitsquelle |
| 12 | Dialysatableitung |
| 122 | Bypassventil |
| 124 | erstes Dialysatorventil, Dialysator-Einlaßventil |
| 125 | zweites Dialysatorventil, Dialysator-Auslaßventil |
| 126 | Dialysatdruckaufnehmer |
| 128 | Blutleckdetektor |
| 144 | Dialysatauslaßleitung, führt vom Dialysator zur Abflußleitung 12 |
| 170 | Zuspritzstelle für Clearancemessung |
| 172 | Erster Sensor zur Clearancemessung |
| 174 | Zweiter Sensor zur Clearancemessung |
| 180 | Erstes Umwegventil (elektromagnetisches Ventil oder Konstantdruckventil) |
| 182 | Zweites Umwegventil (elektromagnetisches oder Rückschlagventil) |
| 184 | Drittes Umwegventil (elektromagnetisches Ventil) |
| 186 | Kartusche für Pulver oder flüssiges Konzentrat |
| 190 | Auswerteeinheit |
| 200 | Dialysator |
| 220 | Blutpumpe |
| 230 | Arterielles Blutschlauchsystem |
| 242 | Venöses Blutschlauchsystem |
| 244 | Venöse Tropfkammer |

(fortgesetzt)

| Liste der Bezeichnungen | |
|---|---|
| Referenznummer | Bezeichnung |
| 246 | Infusionsanschluß an der venösen Tropfkammer |
| 248 | Konnektor am Infusionsanschluß |
| 312 | Durchflußmesser |

**Patentansprüche**

1. Vorrichtung zur Messung von Stoffaustauschparametern in der Hämodialyse und Hämodiafiltration mit

   einem Dialysator (200), der durch eine semipermeable Membran in einen Blutraum und einen Dialysatraum geteilt ist,

   einem den Blutraum des Dialysators einschließenden Blutkreislauf (230, 242), der entweder mit einem Patienten oder einer Flüssigkeitsquelle und einem Flüssigkeitsabfluss verbindbar ist,

   einem den Dialysatraum des Dialysators einschließenden Dialysatkreislauf (10, 12), der einerseits mit einer Dialysat- oder einer Wasserquelle und andererseits mit einem Abfluss verbindbar ist,

   Mitteln (174) zur Bestimmung der Konzentration eines Stoffes im Dialysat stromab des Dialysators,

   Mitteln (170) zur Zugabe des Stoffes, dessen Austauschparameter gemessen werden soll, in das Dialysat als Bolus stromauf des Dialysators,

   **gekennzeichnet durch**,

   eine mit den Mitteln (174) zur Bestimmung der Konzentration des Stoffes im Dialysat und den Mitteln (170) zur Zugabe des Stoffes zusammenwirkende Auswerteinheit (190), die derart ausgebildet ist, dass die Dialysance D aus der stromauf des Dialysators als Bolus zugegebenen Menge des Stoffes und dem Integral über die Zeit der auf den Bolus zurückzuführenden Änderung der Konzentration des Stoffes im Dialysat stromab des Dialysators sowie dem Dialysierflüssigkeitsfluss bestimmbar ist.

2. Vorrichtung nach Anspruch 1, **dadurch gekennzeichnet, dass** die Mittel (170) zur Zugabe eines Stoffes in das Dialysat eine Spritze umfassen.

3. Vorrichtung nach Anspruch 1, **dadurch gekennzeichnet, dass** die Mittel (170) zur Zugabe eines Stoffes in das Dialysat eine Konzentratpumpe umfassen.

4. Vorrichtung nach Anspruch 1, **dadurch gekennzeichnet, dass** die Mittel (170) zur Zugabe eines Stoffes in das Dialysat ein Konzentrat enthaltendes Vorratsgefäß (186) umfassen, das in den Dialysatkreislauf (10, 12) schaltbar ist.

5. Vorrichtung nach einem der Ansprüche 1 bis 4, **dadurch gekennzeichnet, dass** die Mittel (174) zur Bestimmung der Konzentration eines Stoffes einen Leitfähigkeitssensor umfassen.

6. Vorrichtung nach einem der Ansprüche 1 bis 4, **dadurch gekennzeichnet, dass** die Mittel (174) zur Bestimmung der Konzentration eines Stoffes einen optischen Sensor umfassen.

7. Vorrichtung nach Anspruch 6, **dadurch gekennzeichnet, dass** der optische Sensor ein die optische Extinktion im Ultraviolettbereich messender Sensor ist.

8. Vorrichtung nach einem der Ansprüche 1 bis 4, **dadurch gekennzeichnet, dass** die Mittel (174) zur Bestimmung der Konzentration eines Stoffes eine ionensensitive Elektrode umfassen.

**9.** Vorrichtung nach einem der Ansprüche 1 bis 4, **dadurch gekennzeichnet, dass** die Mittel (174) zur Bestimmung der Konzentration eines Stoffes einen optischen Sensor zur Bestimmung der optischen Drehung oder des Brechnungsindex umfassen.

**10.** Vorrichtung nach einem der Ansprüche 1 bis 9, **dadurch gekennzeichnet, dass** der dem Dialysat zugegebene Stoff Wasser ist.

**Revendications**

**1.** Dispositif pour la mesure de paramètres d'échange de substances lors de l'hémodialyse et de l'hémodiafiltration, comportant

un dialyseur (200), qui est divisé par une membrane semi-perméable en une chambre pour le sang et une chambre pour le dialysat,
un circuit pour le sang (230, 242), qui inclut la chambre pour le sang du dialyseur et peut être relié soit à un patient, soit à une source de liquide et à une évacuation pour le liquide,
un circuit à dialysat (10, 12), qui inclut la chambre à dialysat du dialyseur et qui peut être relié d'une part à une source dialysat ou à une source d'eau et d'autre part à une évacuation,
des moyens (174) pour déterminer la concentration d'une substance dans le dialysat en aval du dialyseur,
des moyens (170) pour l'addition d'une substance, dont le paramètre d'échange doit être mesuré, dans le dialysat en tant que bolus en amont du dialyseur,

**caractérisé par**

une unité d'évaluation (190), qui coopère avec les moyens (174) de détermination de la concentration de la substance dans le dialysat et avec les moyens (170) d'ajout d'une substance dans le dialysat, et qui est agencée de telle sorte que la dialysance D peut être déterminée à partir de la quantité de la substance introduite en tant que bolus en aval du dialyseur, et de l'intégrale dans le temps de la variation, imputable au bolus, de la concentration de la substance dans le dialysat en aval du dialyseur ainsi que de l'écoulement de liquide du dialyseur.

**2.** Dispositif selon la revendication 1, **caractérisé en ce que** les moyens (170) d'ajout d'une substance dans le dialysat comprennent une seringue.

**3.** Dispositif selon la revendication 1, **caractérisé en ce que** les moyens (170) d'addition d'une substance dans le dialysat comprennent une pompe à concentré.

**4.** Dispositif selon la revendication 1, **caractérisé en ce que** les moyens (170) d'addition d'une substance dans le dialysat comprennent un réservoir (186) contenant un concentré et qui peut être inséré dans le circuit (10, 12) pour le dialysat.

**5.** Dispositif selon l'une des revendications 1 à 4, **caractérisé en ce que** les moyens (174) pour déterminer la concentration d'une substance comprennent un capteur de conductivité.

**6.** Dispositif selon l'une des revendications 1 à 4, **caractérisé en ce que** les moyens (174) pour déterminer la concentration d'une substance comprennent un capteur optique.

**7.** Dispositif selon la revendication 6, **caractérisé en ce que** le capteur optique est un capteur mesurant l'extinction optique dans le domaine de l'ultraviolet.

**8.** Dispositif selon l'une des revendications 1 à 4, **caractérisé en ce que** les moyens (174) pour déterminer la concentration d'une substance comprennent une électrode sensible aux ions.

**9.** Dispositif selon l'une des revendications 1 à 4, **caractérisé en ce que** les moyens (174) pour déterminer la concentration d'une substance comprennent un capteur optique pour déterminer la rotation optique ou l'indice de réfraction.

**10.** Dispositif selon l'une des revendications 1 à 9, **caractérisé en ce que** la substance ajoutée au dialysat est de l'eau.

**Claims**

**1.** Device for measuring metabolic parameters in haemodialysis and haemodiafiltration, having a dialyser (200) which is divided into a blood space and a dialysate space by a semi-permeable membrane, having a blood circuit (230, 242), which includes the blood space of the dialyser and can be connected either to a patient or to a solution source and a solution drain, having a dialysate circuit (10, 12), which includes the dialysate space of the dialyser and can be connected on the one hand to a source of dialysate or water and on the other hand tn a drain, having means (174) for determining the concentration of a substances in the dialysate downstream of the dialyser, and having means (170) for adding the substance whose metabolic parameter is to be measured to the dialysate as a bolus upstream of the dialyser, **characterised by** an analysing unit (190) which co-operates with the means (174) for determining the concentration of the substances in the dialysate and with the means (170) for adding the substance, which analysing unit (190) is so arranged that the dialysance D is determinable from the quantity of the substance which is added upstream of the dialyser as a bolus, from the integral over time of the change in the concentration of the substance in the dialysate downstream of the dialyser, which change is attributable to the bolus, and from the flowrate of the dialysis solution.

**2.** Device according to claim 1, **characterised in that** the means (170) for adding a substance to the dialysate comprise a syringe.

**3.** Device according to claim 1, **characterised in that** the means (170) for adding a substance to the dialysate comprise a concentrate pump.

**4.** Device according to claim 1, **characterised in that** the means (170) for adding a substance to the dialysate comprise a supply vessel (186) containing concentrate which can be connected into the dialysate circuit (10, 12).

**5.** Device according to one of claims 1 to 4, **characterised in that** the means (174) for determining the concentration of a substance comprise a conductivity sensor.

**6.** Device according to one of claims 1 to 4, **characterised in that** the means (174) for determining the concentration of a substance comprise an optical sensor.

**7.** Device according to claim 6, **characterised in that** the optical sensor is a sensor which measures optical extinction in the ultraviolet range.

**8.** Device according to one of claims 1 to 4, **characterised in that** the means (174) for determining the concentration of a substance comprise an ion-sensitive electrode.

**9.** Device according to one of claims 1 to 4, **characterised in that** the means (174) for determining the concentration of a substance comprise an optical sensor for determining optical rotation or the index of refraction.

**10.** Device according to one of claims 1 to 9, **characterised in that** the substance added to the dialysate is water.

**Figur 1**

cBo

cDi
QD

200

cDo

QB | cBi

**Figur 2a**

$$\Delta Mi$$

$$cDi(1) = cDi(0) + dcDi$$

$$cDi(0)$$

$$\Delta Mo$$

$$cDo(1) = cDo(0) + dc\Gamma$$

$$cDo(0)$$

c

t, Q*t

**Figur 2b**

**Figur 3**